# EUROPEAN PATENT SPECIFICATION

(11) **EP 3 815 667 B1**
(45) Date of publication and mention of the grant of the patent: **03.01.2024**
(21) Application number: 19825892.3
(22) Date of filing: 27.06.2019
(51) Int. Cl.: A61H 19/00, A61F 5/41, A61F 5/453, A61F 5/451, A61H 23/02

(54) **DEVICE FOR PRACTISING EJACULATORY CONTROL**
GERÄT ZUR ÜBUNG DER EJAKULATIONSKONTROLLE
DISPOSITIF POUR EXERCER LE CONTRÔLE DE L'ÉJACULATION

(30) Priority: 27.06.2018 ES 201830996 U
(43) Date of publication of application: 05.05.2021
(73) Proprietor: New Wellness Concept, S.L., 41010 Sevilla (ES)
(72) Inventor: LÓPEZ TRABAJO, Patricia, 41010 Sevilla (ES)
(74) Representative: Pons
(86) International application number: PCT/ES2019/070451
(87) International publication number: WO 2020/002738

(56) References cited:
- WO-A1-2011/067590
- WO-A1-2014/053122
- CN-A- 102 657 542
- CN-U- 202 005 872
- ES-T3- 2 380 048
- JP-A- 2010 142 577
- US-A1- 2010 041 944
- US-A1- 2012 136 203
- US-A1- 2012 215 189
- US-A1- 2012 215 189
- US-A1- 2014 135 650
- US-A1- 2015 034 504
- US-A1- 2016 199 214
- US-A1- 2018 021 213

## Description

### OBJECT OF THE INVENTION

The invention, as expressed in the wording of the present specification, refers to a device to exercise male ejaculatory control that provides, to the function for which it is intended, advantages and characteristics, which are described in detail below and which involve a novelty in the current state of the art.

The object of this invention lies in an electrical actuation device that, formed from a receptacle to receive the male erect penis, is structurally designed to provide a practical and effective means of stimulation to exercise the user's ejaculatory control, distinguishing itself by comprising an interior configuration that faithfully emulates the morphology of a vaginal canal, a means to heat the wall of said interior through a special cover, and a means for vibration with specific location.

### FIELD OF APPLICATION OF THE INVENTION

The field of application of this invention is framed within the sector of the industry dedicated to the manufacture of medical apparatuses and devices, focusing particularly on the field of those intended for problems with ejaculatory control, at the same time covering the field of male stimulators.

### BACKGROUND OF THE INVENTION

As is known, premature ejaculation (PE) is one of the most common male sexual dysfunctions. Despite new criteria and classifications in recent years for a better definition of this disease, the true prevalence of PD remains unclear.

Regarding its treatment, the evidence to date confirms the efficacy and safety of dapoxetine (LOE "level of evidence" 1a), selective serotonin reuptake inhibitors (SSRIs) offlabel (LOE 1a) and anesthetics for topical use (LOE 1b). According to the International Society for Sexual Medicine (ISSM), these are considered the first choice for its treatment.

In relation to conductive treatments (CTs), the evidence is very limited (LOE 2b), despite the fact that there is an improvement in the results when drugs are combined with CT, in contrast to the exclusive use of drugs.

A new line of CTs has arisen from the technological development and worldwide popularity reached by the sex toy industry in recent years. These treatment programs combine classical behavioral techniques such as the Semans stop and start with the use of devices to aid masturbation.

In 2000, a first article by Wise et al., in which reference was made to the use of a vibratory desensitizing device to aid masturbation in the treatment of PD in a series of cases, where the mean increase in the Intravaginal Ejaculatory Latency Time (TLEI) per patient was 315% after six weeks of use.

However, it is not a masturbator, but a flat vibrating device that is placed on the glans to desensitize the area. Unlike this, the device of this invention does not try to desensitize, but rather for the patient to acquire control of the moment of ejaculation through exercises that allow you to relax the sphincter of the urethra, which is directly related to the moment of ejaculation.

The device designed by Zamar a year earlier specially to desensitize was based on the theory of hypersensitivity of the penis as a cause of PD. Its designer considered that an increase in the intensity and frequency of stimulation of a hypersensitive organ resulted in a habituation effect.

In 2012, it was Zamar himself who, at the World Congress of Sexual Medicine in Chicago, presented a clinical trial with 56 participants in which he repeated the methodology of Wise, et al., but with a new version of his own vibrating device to aid masturbation and in which he argued for combining with the Semans stop-start technique as an enhancing element of the device. In this study, 61% of the patients increased their TLEI an average of 11 points.

Two years later Jem would replicate this Zamar clinical trial with a sample of 13 subjects. As a novelty, a follow-up was done at 3 and 6 months after the end of the treatment. The author indicates the impossibility of finding differences as significant as Zamar between the control group and the waiting list group in the TLEI, although he acknowledges that there are detectable improvements and that these would remain at 3 and 6 months.

Rodriguez et al., presented a series of cases replicating the study by Wise et al., in which, as a novelty, a commercial device to aid masturbation marketed as a sex toy was used, other than 2 their conclusions.

The results indicated increases in TLEI of between 63% and 92%. In this case, the researchers explain their results based on a process of habituation of the glans receptors due to repeated use and the intense stimulation of nerve receptors caused by the device, in line with the postulates of Wise, et al.

A year later, Rodriguez et al., published another series of cases in which a new device for commercial use is used with slight modifications to that of their previous study.

Of the 20 cases initially included in the study, 18 post-treatment measures were valid. The methodology was improved, and a clinical improvement criterion was established based on the pre- and post-treatment results of the Premature Ejaculation Profile (PER), instead of using the measurement of the TLEI. A very important percentage of men met the established criteria for clinical benefit, 83% in control over ejaculation, 72% in stress and interpersonal difficulties associated with ejaculation, and 33% in satisfaction with their coital relationships.

In this case, the authors explain the improvements found by using the device for 6 weeks, together with the combination of the stop-and-go strategy established in the rules of use, thus abandoning the hypothesis of habituation (desensitization). They consider that training ejaculatory control with a stimulation similar to that given during intercourse in the receptors of the glans, favors the generalization of what is learned during masturbation to intercourse.

A recent review of this new line of treatment for the use of devices to aid masturbation in combination with behavioral techniques considers this therapeutic approach as a promising alternative to pharmacological treatments, which could not only obtain better TLEI but also provide control to men with PD, without side effects and with stable results over time.

The objective of this invention is, therefore, to develop a stimulation device to facilitate the practice of such exercises, the characteristics of which make it optimal for this.

On the other hand, and as a reference to the current state of the art, it should be noted that, although a device for exercising ejaculatory control with a similar structure and operation is known on the market, at least by the applicant, the existence according to any presenting technical and structural characteristics equal to those presented by the one claimed herein is unknown.

In this sense, it should be mentioned that the devices of this type on the market, in addition to not being designed as a therapeutic instrument to carry out ejaculation control exercises, usually present configuration and heat and vibration functions that are far from simulating a vagina in a state of excitement, which causes its effectiveness to be very limited, regardless of the use that is given.

For example, Document US 2010/041944A discloses the features according to the preamble of independent claim 1 of the present invention. More in particular, it discloses a stimulation device comprising vibrations means in the distal end of an inner canal, said vibration means working at frequencies ranging from low (zero Hz) to very high (1000Hz), for example, varying randomly.

Document US 2015/034504 discloses a human stimulation device according to the preamble of independent claim 1.

Document WO2014/053122A describes a a device with massage and intensity control, wherein the massage movement is applied in the distal end by means of vibratory motors.

Document US 2018/021213 describes a male stimulation device wherein an insert or shirt that receives the human part may comprises vibration, removable vibration and/or infrared in the whole area covered.

Document US 2012/215189A, describes sperm collector with squeezing function wherein at least two symmetrically placed vibration means are placed in the distal thicker end of the device.

### EXPLANATION OF THE INVENTION

The device for exercising ejaculatory control proposed by the invention is therefore configured as a remarkable novelty within its field of application, the characterizing details that distinguish it being conveniently collected in the final claims that accompany this specification.

Specifically, what the invention proposes, as indicated above, is an electrical actuation device that comprises a receptacle to receive the male erect penis, which is structurally designed to provide a practical and effective means of stimulation for the purpose of serving as a system to exercise the user's ejaculatory control.

To do this, said device is essentially distinguished by comprising a base forming such a receptacle internally equipped with an interior channel whose configuration faithfully emulates the morphology of a vaginal canal, a heated means of the wall of said interior in the form of a vaginal canal through a removable and specially designed cover, and a means of vibration with specific location and frequency.

Specifically, to realistically recreate the vaginal introitus and reproduce it in the aforementioned inner canal, a file was taken as an example that reproduced a sagittal section of a real vagina. From this document, and aided by the indications of a group of sexologists, a canal was reproduced, which simulates the pressure at the entrance of the vagina, to its roughness and protrusions, such as that of the pubic bone, so that the sensation when penetrating the device is as real as possible and similar to the sensation of penetrating a real vagina. With this, therefore, said inner canal presents a narrow initial zone, an intermediate zone with roughness and protuberances and a thickened final end similar to those of a real vagina.

Specifically, and in order to simulate a vagina in a state of excitement, preferably said inner canal has a narrow initial area, to thus simulate the pressure of the pubic bone on the penis at the moment of penetration, an intermediate area with roughness and bumps that simulate the different muscular layers of the vagina and a thickened end that is swells slightly, similar to those of a real vagina in the part that adjoins the uterus.

The vagina is a flexible fibromuscular canal. The length of the vagina in humans varies from 8 to 10 cm on average. It has a length of 7 cm on its front face, while the back, which is the longest, measures 9 cm. However, when sexually stimulated, the measurements increase to 12 centimeters. The vaginal diameter in an unstimulated vagina is 2 centimeters, but at the time of orgasm it can reach 6 centimeters. For this reason, the internal canal is preferably made of a thermoplastic material, which allows said expansion during penetration, due to its flexibility. Preferably, the diameter of the channel of the device is 2 cm (expandable to 6 cm) and has a length of 12 centimeters, thus simulating a vagina in excitement.

Going into more detail, it should be noted that the base of the device is made up of an external casing, preferably made of plastic or another rigid and resistant material, for example, acrylonitrile butadiene styrene to ABS which is a plastic that is very resistant to impact, and for an internal piece of soft thermoplastic material that defines the aforementioned internal canal in the form of a vaginal canal, covering most of the interior of the external casing and extending to the opening.

Meanwhile, the removable cover constituting the heating means of the inner canal wall, has a lower heating rod through which, when inserted into the inner canal of the base, heat is transferred from said canal to the wall, with which, in addition to reproducing the heat zone in a more realistic way, it is also possible to carry out said heating very quickly and with greater temperature control, which preferably oscillates between 36.5 and 37°C, which is the normal temperature that the human body usually has, avoiding excessive overheating that can be counterproductive for the achievement of the stimulation objective.

Another of the unmistakable characteristics of a real penetration is the sensation of inserting the penis in a humid and warm environment. To recreate the humidity, the treatment contemplates the use of a lubricant, and to recreate the temperature, the aforementioned heating rod has been incorporated into the aforementioned cover, that is inserted into the inner canal of the base when placing the cover and connects to the current of the base itself, heating its interior until it reaches body temperature (36.5/37 degrees), at which point an indicator light built into the temperature button of the case, turns on/off, alerting the user that the device has warmed up to body temperature and is ready for use in approximately 10 minutes.

Regarding the means of vibration, these are determined by the existence of a vibrating motor that is located 5 centimeters from the entrance of the vaginal canal and works at a frequency of amplitude 2.5mm and 100HZ, since, as has been said, it has a specific location and frequency that are an integral part of the design of the device, which are determined based on the conclusions of a new line of research that uses Vibratory Penis Stimulation (VPS), as a therapeutic method for spinal cord injuries, whose "gold standard" establishes that said amplitude of 2.5 mm and 100 HZ to be applied in the area of the frenulum of the glans. Therefore, the point of vibration is 5 cm from the entrance of the vaginal canal and must be located in the lower part of the penis. The device must always be used in the same position during the masturbation exercise (its ergonomic design facilitates this position), so that the vibrating point stimulates the lower ventral part of the penis, more specifically the frenulum glans as before mentioned, when typical masturbation movements are being performed with it.

Finally, it should be noted that the device incorporates at its base a power battery housed inside the housing, which is preferably rechargeable and is linked to a charging point with a magnet, suitable for coupling to a recharging support that prevents the existence of external connection wiring or the need to replace batteries.

The described device for exercising ejaculatory control consists therefore of an innovative structure with characteristics unknown until now for the purpose for which it is intended, reasons that, together with its practical utility, provide it with a sufficient basis to obtain the privilege of exclusivity requested.

### DESCRIPTION OF THE DRAWINGS

To complement the description being made and for the purpose of helping to provide better comprehension of the invention's characteristics, as an integral part of said description, a plan in which the following has been represented for illustrative and non-limiting purposes:
Figure number 1.- Shows a schematic cross section view of the side elevation of an example of an embodiment of the device to exercise dismantled ejaculatory control (with the cover removed), object of the invention, showing the main parts and elements that it comprises, as well as the configuration and arrangement thereof.
Figure number 2.- Shows a schematic cross section view of the side elevation of an example of an embodiment of the device to exercise ejaculatory control mounted (with the cover in place), object of the invention, showing the main parts and elements that it comprises, as well as the configuration and arrangement thereof.

### PREFERRED EMBODIMENT OF THE INVENTION

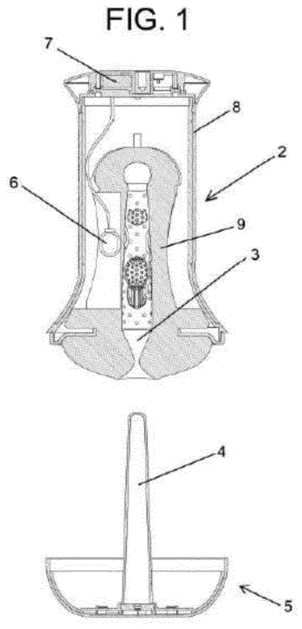

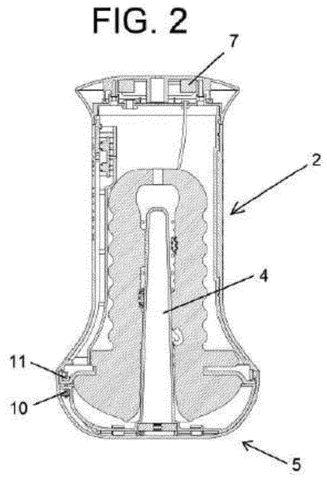

In view of Figure 1 and 2 described, and in accordance with the numbering adopted, an example of a non-limiting embodiment of the device for exercising ejaculatory control of the invention can be seen, which comprises the parts and elements indicated and described in detail below.

Thus, as can be seen in said figure, the device (1) in question is essentially comprised of: a base (2) forming a receptacle that has an inner canal (3) with a configuration provided with a narrow initial zone, an intermediate zone with roughness and bumps and a thickened final end that faithfully emulates the morphology of a vaginal canal; heated means (4) that transfer heat to the wall of said inner canal (3) incorporated in a removable cover (5); vibratory means (6) with specific location near the end of the inner channel (3) and with a working frequency, also specific, of an amplitude of 2.5mm and 100HZ; and an electrical power supply means (7).

Preferably, the base (2) of the device is formed by an external casing (8), made of ABS or similar material, and an internal piece (9) of soft thermoplastic material that defines the inner channel (3) in the form of a vaginal canal, which covers most of the inside of the outer casing (8) and it extends to the opening where it has its entrance, upon which the cover (5) fits.

Preferably, the heating means (4) contained in that said cover (5) are determined by a lower heating rod that, when placing the cover (5) upon the base (2), is inserted into the inner canal (3) thereof. And, preferably, a temperature of between 36.5 to 37°C will be reached.

Said heating means (4) are coupled to a connector (10) provided in the cover (5) that contacts a complementary connector (11) of the base (2) when said cover (5) is coupled to the base (2), which, in turn, is connected to the electrical power supply means (7) located at the base (2) and consisting, preferably, of a rechargeable battery.

The base (2) incorporates a temperature button with an indicator light connected to the heating means (4), through the aforementioned connector (10) of the cover (5) and the complementary connector (11) of the base (2), to activate and witness its activation.

Preferably, the base (2) also incorporates a vibration button that, through a third connector, is linked to the motor that defines the vibratory means (6) to activate its drive.

The rechargeable battery that defines the electrical power supply means (7) is linked to a recharging point with a magnet, suitable for attachment to a charging stand.

Sufficiently described the nature of this invention, as well as the manner in which to put it into practice, it is not considered necessary to make its explanation more extensive so that any expert in the field may understand its scope and the advantages derived therefrom, stating that, within its essentiality, it may be taken to practice in other embodiments that differ in details to that indicated as an example, and to which will also be granted the protection sought, provided that it does not alter, change or modify its fundamental principle.

## Claims

1. Device to exercise ejaculatory control, formed from a base (2) provided with an inner canal (3) to receive the erect male penis, vibratory means (6), and electrical power supply means (7), wherein the inner canal (3) has an entrance next to a narrower proximal zone, an intermediate zone with roughness and bumps and a thicker distal end; and **characterized in that** the vibratory means (6) are located at 5 cm from the entrance of the inner canal (3) and said vibratory means work at a frequency of 100HZ and at an amplitude of 2,5 mm, intended to be applied, during use, in the area of the frenulum of the glans .

2. Device to exercise ejaculatory control, according to claim 1, **characterized in that** it comprises a removable cover (5) with heated means (4) that transfer heat to the wall of said inner canal (3).

3. Device to exercise ejaculatory control, according to claim 2, **characterized in that** the heating means (4) which has the removable cover (5) are controlled by a lower heating rod that, when placing the cover (5) upon the base (2), it is inserted into the inner canal (3) thereof.

4. Device to exercise ejaculatory control, according to claims 2 to 3, **characterized in that** the heating means (4) are coupled to a connector (10) provided in the cover (5) that contacts a complementary connector (11) of the base (2) when said cover (5) is coupled to the base (2), which, in turn, is connected to the electrical power supply means (7) located at the base (2).

5. Device to exercise ejaculatory control, according to claim 4, **characterized in that** the base (2) incorporates a temperature button with an indicator light connected to the heating means (4), through the aforementioned connector (10) of the cover (5) and the complementary connector (11) of the base (2), to activate and give evidence of its activation.

6. Device to exercise ejaculatory control, according to any of claims 2-5, **characterized in that** the heating means (4) reach a temperature of between 36.5 to 37°C.

7. Device to exercise ejaculatory control, according to any of the foregoing claims **characterized in that** the inner canal (3) is made of a thermoplastic material and the diameter of the inner canal (3) is 2 cm expandable to 6 cm and has a length of 12 centimeters.

8. Device to exercise ejaculatory control, according to any of the foregoing claims, **characterized in that** the external shape of the device (1) shows the user the position in which to use the device.

## Patentansprüche

1. Vorrichtung zur Ausübung der Ejakulationssteuerung, gebildet aus einer Basis (2), die mit einem inneren Kanal (3) zur Aufnahme des erigierten männlichen Penis, Vibrationsmitteln (6) und elektrischen Energieversorgungsmitteln (7) versehen ist, wobei der innere Kanal (3) einen Eingang neben einer schmaleren proximalen Zone, eine Zwischenzone mit Unebenheit und Erhebungen und ein dickeres distales Ende aufweist; und **dadurch gekennzeichnet, dass** die Vibrationsmittel (6) 5 cm vom Eingang des inneren Kanals (3) gelagert sind und die Vibrationsmittel mit einer Frequenz von 100 Hz und einer Amplitude von 2,5 mm arbeiten, dazu vorgesehen, während des Gebrauchs im Bereich des Frenulums der Eichel eingesetzt zu werden.

2. Vorrichtung zur Ejakulationssteuerung gemäß Anspruch 1, **dadurch gekennzeichnet, dass** sie eine abnehmbare Abdeckung (5) mit beheizten Mitteln (4), die Wärme an die Wand des inneren Kanals (3) übertragen, umfasst.

3. Vorrichtung zur Ausübung der Ejakulationssteuerung gemäß Anspruch 2, **dadurch gekennzeichnet, dass** die Heizmittel (4), die die abnehmbare Abdeckung (5) aufweist, durch eine untere Heizstange gesteuert werden, die beim Aufsetzen der Abdeckung (5) auf die Basis (2) in den inneren Kanal (3) davon eingeführt wird.

4. Vorrichtung zur Ausübung der Ejakulationssteuerung gemäß den Ansprüchen 2 bis 3, **dadurch gekennzeichnet, dass** die Heizmittel (4) an ein in der Abdeckung (5) vorgesehenes Verbindungselement (10) gekoppelt sind, das, wenn die Abdeckung (5) an die Basis (2) gekoppelt wird, mit einem komplementären Verbindungselement (11) der Basis (2) in Kontakt tritt, das wiederum an die elektrischen Energieversorgungsmittel (7), die an der Basis (2) gelagert sind, angeschlossen ist.

5. Vorrichtung zur Ausübung der Ejakulationssteuerung gemäß Anspruch 4, **dadurch gekennzeichnet, dass** die Basis (2) einen Temperaturknopf mit einer Anzeigenleuchte enthält, der über das oben genannte Verbindungselement (10) der Abdeckung (5) und das komplementäre Verbindungselement (11) der Basis (2) an das Heizmittel (4) angeschlossen ist, um aktiviert zu werden und seine Aktivierung zu belegen.

6. Vorrichtung zur Ausübung der Ejakulationssteuerung gemäß einem der Ansprüche 2 bis 5, **dadurch gekennzeichnet, dass** die Heizmittel (4) eine Temperatur zwischen 36,5 und 37 °C erreichen.

7. Vorrichtung zur Ausübung der Ejakulationssteuerung gemäß einem der vorhergehenden Ansprüche, **dadurch gekennzeichnet, dass** der innere Kanal (3) aus einem thermoplastischen Material besteht und der Durchmesser des inneren Kanals (3) 2 cm beträgt, auf 6 cm erweiterbar ist und eine Länge von 12 Zentimetern aufweist.

8. Vorrichtung zur Ausübung der Ejakulationssteuerung gemäß einem der vorhergehenden Ansprüche, **dadurch gekennzeichnet, dass** die äußere Form der Vorrichtung (1) dem Benutzer die Position anzeigt, in der er die Vorrichtung verwenden soll.

## Revendications

1. Dispositif pour exercer le contrôle de l'éjaculation, formé à partir d'une base (2) pourvue d'un canal interne (3) pour recevoir le pénis masculin en érection, d'un moyen de vibration (6) et d'un moyen d'alimentation électrique (7), dans lequel le canal interne (3) possède une entrée à côté d'une zone proximale plus étroite, une zone intermédiaire dotée d'une rugosité et de protubérances et une extrémité distale plus épaisse ; et **caractérisé en ce que** le moyen de vibration (6) est situé à 5 cm de l'entrée du canal interne (3) et ledit moyen de vibration fonctionne à une fréquence de 100 Hz et à une amplitude de 2,5 mm, destiné à être appliqué, pendant l'utilisation, dans la zone du frein du gland.

2. Dispositif pour exercer le contrôle de l'éjaculation, selon la revendication 1, **caractérisé en ce qu'**il comprend un couvercle amovible (5) doté d'un moyen chauffé (4) qui transfère de la chaleur à la paroi dudit canal interne (3).

3. Dispositif pour exercer le contrôle de l'éjaculation, selon la revendication 2, **caractérisé en ce que** le moyen de chauffage (4) que possède le couvercle amovible (5) est contrôlé par une tige de chauffage inférieure qui, lors du placement du couvercle (5) sur la base (2), est insérée dans le canal interne (3) de celle-ci.

4. Dispositif pour exercer le contrôle de l'éjaculation, selon les revendications 2 à 3, **caractérisé en ce que** le moyen de chauffage (4) est couplé à un connecteur (10) pourvu dans le couvercle (5) qui vient en contact avec un connecteur complémentaire (11) de la base (2) lorsque ledit couvercle (5) est couplé à la base (2), lequel, à son tour, est connecté au moyen d'alimentation électrique (7) situé au niveau de la base (2).

5. Dispositif pour exercer le contrôle de l'éjaculation, selon la revendication 4, **caractérisé en ce que** la base (2) intègre un bouton de température doté d'un voyant lumineux connecté au moyen de chauffage (4), par le biais du connecteur (10) susmentionné du couvercle (5) et du connecteur complémentaire (11) de la base (2), pour l'activer et témoigner de son activation.

6. Dispositif pour exercer le contrôle de l'éjaculation, selon l'une quelconque des revendications 2 à 5, **caractérisé en ce que** le moyen de chauffage (4) atteint une température comprise entre 36,5 et 37 °C.

7. Dispositif pour exercer le contrôle de l'éjaculation, selon l'une quelconque des revendications précédentes, **caractérisé en ce que** le canal interne (3) est constitué d'un matériau thermoplastique et le diamètre du canal interne (3) est de 2 cm extensible à 6 cm et possède une longueur de 12 centimètres.

8. Dispositif pour exercer le contrôle de l'éjaculation, selon l'une quelconque des revendications précédentes, **caractérisé en ce que** la forme externe du dispositif (1) montre à l'utilisateur la position dans laquelle utiliser le dispositif.
